(19) Europäisches Patentamt
European Patent Office
Office européen des brevets

(11) EP 1 542 647 B1

(12) **EUROPÄISCHE PATENTSCHRIFT**

(45) Veröffentlichungstag und Bekanntmachung des Hinweises auf die Patenterteilung:
**24.09.2014 Patentblatt 2014/39**

(21) Anmeldenummer: **03794904.7**

(22) Anmeldetag: **21.08.2003**

(51) Int Cl.:
***A61K 8/49*** *(2006.01)* ***A61Q 5/10*** *(2006.01)*

(86) Internationale Anmeldenummer:
**PCT/EP2003/009249**

(87) Internationale Veröffentlichungsnummer:
**WO 2004/024109 (25.03.2004 Gazette 2004/13)**

(54) **Verwendung von Färbemitteln mit 5,6-Dihydroxyindolin oder seinen physiologisch verträglichen Salzen**

Use of hair colouring products comprising 5,6-dihydroxyindoline or a physiologically acceptable salt thereof

Utilisation de produits de coloration capillaire comprenant 5,6-dihydroxyindoline ou l'un de ses sels physiologiquement acceptable

(84) Benannte Vertragsstaaten:
**AT BE BG CH CY CZ DE DK EE ES FI FR GB GR HU IE IT LI LU MC NL PT RO SE SI SK TR**

(30) Priorität: **30.08.2002 DE 10240758**

(43) Veröffentlichungstag der Anmeldung:
**22.06.2005 Patentblatt 2005/25**

(73) Patentinhaber: **Henkel AG & Co. KGaA**
**40589 Düsseldorf (DE)**

(72) Erfinder:
- **AKRAM, Mustafa**
**22457 Hamburg (DE)**
- **HÖFFKES, Horst**
**40595 Düsseldorf (DE)**
- **RATH, Susanne**
**20359 Hamburg (DE)**

(56) Entgegenhaltungen:
**EP-A- 0 728 464** **WO-A-01/93819**
**WO-A-93/09759** **DE-A- 4 139 675**
**DE-A- 10 037 158** **DE-A- 10 120 915**
**DE-U- 20 118 089**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 542 647 B1

Printed by Jouve, 75001 PARIS (FR)

## Beschreibung

Die vorliegende Erfindung betrifft die Verwendung von 5,6-Dihydroxyindolin oder einem physiologisch verträglichen Salz hiervon zur Verbesserung des Egalisierungsvermögens von Färbemitteln.

Für das Färben von Keratinfasern, insbesondere menschlichen Haaren, spielen die sogenannten Oxidationsfarbemittel wegen ihrer intensiven Farben und guten Echtheitseigenschaften eine bevorzugte Rolle. Solche Färbemittel enthalten Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehrerer Kupplerkomponenten die eigentlichen Farbstoffe aus.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis(2'-hydroxyethyl)-p-phenylendiamin, 2-(2',5'-Diaminophenyl)-ethanol, 2-(2',5'-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxy-4,5,6-triaminopyrimidin, 2,4-Dihydroxy-5,6-diaminopyrimidin, 2,5,6-Triamino-4-hydroxypyrimidin und 1,3-N,N'-Bis(2'-hydroxyethyl)-N,N'-bis(4'-aminophenyl)-diamino-propan-2-ol.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin und 2-Methyl-4-chlor-5-aminophenol.

Allein mit einer Entwicklerkomponente oder einer speziellen Kuppler/Entwicklerkombination gelingt es in der Regel nicht, eine auf dem Haar natürlich wirkende Farbnuance zu erhalten. In der Praxis werden daher üblicherweise Kombinationen verschiedener Entwickler- und/oder Kupplerkomponenten eingesetzt. Es besteht daher ständig Bedarf an neuen, verbesserten Farbstoffkomponenten.

Gute Oxidationsfarbstoffvorprodukte sollen in erster Linie folgende Voraussetzungen erfüllen: Sie müssen bei der oxidativen Kupplung die gewünschten Farbnuancen in ausreichender Intensität und Echtheit ausbilden. Sie sollen beständig sein gegen Licht, Wärme, Schweiß, Reibung und den Einfluss chemischer Reduktionsmittel, z.B. Dauerwellenflüssigkeiten. Weiterhin sollen sie - falls als Haarfärbemittel zur Anwendung kommend - die Kopfhaut nicht zu sehr anfärben, und vor allem sollen sie in toxikologischer und dermatologischer Hinsicht unbedenklich sein. Schließlich soll die erzielte Färbung durch Blondierung leicht wieder aus dem Haar entfernt werden können, falls sie doch nicht den individuellen Wünschen der einzelnen Person entspricht und rückgängig gemacht werden soll.

Eine weitere für Färbemittel wesentliche Eigenschaft ist das sogenannte Egalisierungsvermögen. Unter diesem Begriff wird die Eigenschaft eines Färbemittels verstanden, dass bei dessen Auftragung auf die Fasern die Farbstoffvorprodukte beziehungsweise die Kombinationen der Farbstoffvorprodukte gleichmäßig auf die Fasern aufzuziehen, wobei insbesondere bei menschlichen Haaren keine merklichen Unterschiede zwischen strapaziertem (Längen) und frisch nachgewachsenem (Ansatz) Haar bestehen dürfen.

Aufgabe der vorliegenden Erfindung war es daher, oxidative Färbemittel hinsichtlich ihrer färberischen Eigenschaften, insbesondere hinsichtlich ihres Egalisierungsvermögens zu verbessern.

Überraschenderweise wurde nun gefunden, dass durch gemeinsame Verwendung von 5,6-Dihydroxyindolin oder einem physiologisch verträglichen Salz hiervon und mindestens einer Entwicklerkomponente und gegebenenfalls mindestens einer Kupplerkomponente das Egalisierungsvermögen eines oxidativen Färbemittel wesentlich verbessert werden kann.

Gegenstand der vorliegenden Erfindung ist daher die Verwendung von 5,6-Dihydroxyindolin oder einem physiologisch verträglichen Salz hiervon zur Verbesserung des Egalisierungsvermögens von Färbemitteln für keratinische Fasern, die mindestens eine Entwicklerkomponente und gegebenenfalls mindestens eine Kupplerkomponente enthalten, wobei das Indolinderivat gemeinsam mit den übrigen Farbstoffvorprodukten auf die Fasern aufgetragen wird. Unter keratinischen Fasern sind dabei erfindungsgemäß Pelze, Wolle, Federn und insbesondere menschliche Haare zu verstehen.

Im Rahmen der vorliegenden Erfindung werden die Fasern mit einem Färbemittel behandelt, das neben 5,6-Dihydroxyindolin oder einem physiologisch verträglichen Salz hiervon mindestens eine Entwicklerkomponente und gegebenenfalls mindestens eine Kupplerkomponente enthält. Im Anschluss an diese Behandlung können die Fasern gegebenenfalls in üblicherweise gespült werden. Hinsichtlich der erfindungsgemäß bevorzugt einsetzbaren Entwicklerkomponenten und gegebenenfalls Kupplerkomponenten sei an dieser Stelle auf die später im Text folgenden Ausführungen verwiesen.

Der Einsatz von Indol- und/oder Indolinderivaten in der oxidativen Haarfärbung zur Verbesserung der Waschechtheit und der Grauabdeckung wurde bereits in der internationalen Anmeldung WO-A1-93/09759 beschrieben. Dieser Schrift sind aber keinerlei Hinweise auf das verbesserte Egalisierungsvermögen zu entnehmen. Ferner offenbart die WO-A1-01/93819 ein Verfahren zur oxidativen Färbung keratinischer Fasern, bei dem die Fasern mit einem Mittel, enthaltend Indol- und/oder Indolinderivate, unmittelbar vor der eigentlichen Färbung in einem ersten Schritt vorbehandelt werden, um die Waschechtheit und das Egalisierungsvermögen zu verbessern. Im Rahmen der nunmehr beanspruchten Verwendung kann eine Verbesserung des Egalisierungsvermögens der Färbemittel bereits in einem einstufigen - und somit leichter ausführbaren - Verfahren erzielt werden.

5,6-Dihydroxyindolin ist das Indolinderivat der Formel (Ia)

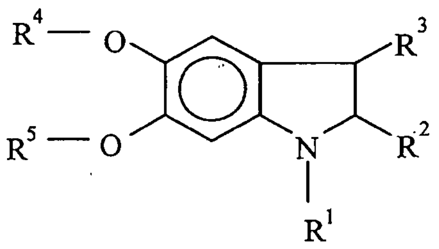

(Ia)

in der unabhängig voneinander

$R^1$ steht für Wasserstoff,
$R^2$ steht für Wasserstoff
$R^3$ steht für Wasserstoff
$R^4$ steht für Wasserstoff,
$R^5$ steht für die unter $R^4$ genannten Gruppe,
oder physiologisch verträgliche Salze dieser Verbindung.

Da es sich bei allen erfindungsgemäßen Substanzen um Amino-Verbindungen handelt, lassen sich aus diesen in üblicher Weise die bekannten Säureadditionssalze herstellen; die Aussagen dieser Schrift und somit der Schutzbereich erstrecken sich somit sowohl auf die Verbindung in freier Form als auch auf deren Salze. Beispiele für solche Salze sind die Hydrochloride, die Hydrobromide, die Sulfate, die Phosphate, die Acetate, die Propionate, die Citrate und die Lactate. Die Hydrobromide können erfindungsgemäß besonders bevorzugt sein.

Die Verbindungen der Formel (Ia) ist 5,6-Dihydroxyindolin.

Das Indolin-Derivat wird bevorzugter Weise in Mengen von 0,01-10 Gew.-%, besonders vorzugsweise von 0,05-5 Gew.% verwendet.

Es kann erfindungsgemäß bevorzugt sein, das 5,6-Dihydroxyindolin oder sein physiologisch verträgliches Salz in Kombination mit mindestens einer Aminosäure oder einem Oligopeptid zu verwenden. Die Aminosäure ist vorteilhafterweise eine $\alpha$-Aminosäure; ganz besonders bevorzugte $\alpha$-Aminosäuren sind Arginin, Ornithin, Lysin, Serin und Histidin, insbesondere Arginin.

Das zu verwendende Mittel enthält 5,6-Dihydroxyindolin oder eines seiner physiologisch verträglichen Salze, insbesondere das Hydrobromid des 5,6-Dihydroxyindolins Eine bevorzugte Verwendung ist dadurch gekennzeichnet, dass das Färbemittel mindestens ein 4,5-Diaminopyrazolderivat enthält.

Weiterhin ist es bevorzugt, wenn es sich bei dem Pyrazolderivat um ein 4,5-Diaminopyrazolderivat handelt. Erfindungsgemäß besonders bevorzugt sind die 4,5-Diaminopyrazolderivate der Formel (II)

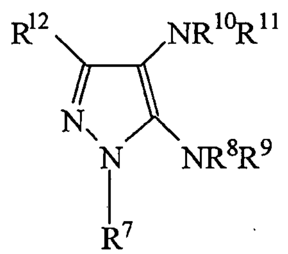

(II)

worin

$R^7$, $R^8$, $R^9$, $R^{10}$ und $R^{11}$ unabhängig voneinander stehen für Wasserstoff, eine geradkettige oder verzweigte $C_{1-6}$-Alkylgruppe, eine $C_{2-4}$-Hydroxyalkylgruppe, eine $C_{2-4}$-Aminoalkylgruppe, eine Phenylgruppe, die gegebenenfalls mit

einer Nitrogruppe, einer Trifluormethylgruppe, einer Aminogruppe oder einer $C_{1-4}$-Alkylaminogruppe substituiert ist, eine Benzylgruppe, die gegebenenfalls mit einem Halogenatom, einer $C_{1-4}$-Alkylgruppe, einer $C_{1-4}$-Alkoxygruppe, einer Methylendioxygruppe oder einer Aminogruppe substituiert ist, eine Pyridylgruppe, eine Thienylgruppe, eine Furylgruppe oder eine Gruppe

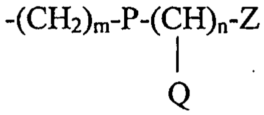

,

worin m und n ganze Zahlen bedeuten, die identisch oder voneinander verschieden sind und im Bereich von 1 bis einschließlich 3 liegen, P steht für Sauerstoff oder eine Gruppe NH, Q steht für Wasserstoff oder Methyl und Z steht für Methyl, eine Gruppe OR oder NRR', worin R und R', die identisch oder voneinander verschieden sein können, Wasserstoff, Methyl oder Ethyl bedeuten,
wobei wenn $R^8$ Wasserstoff bedeutet, $R^9$ auch $C_{1-4}$-Alkylamino bedeuten kann, und
$R^{12}$ steht für eine geradkettige oder verzweigte $C_{1-6}$-Alkylgruppe, $C_{1-4}$-Hydroxyalkylgruppe, $C_{1-4}$-Aminoalkylgruppe, $C_{1-4}$-Alkyl-$C_{1-4}$-aminoalkylgruppe, $C_{1-4}$-Dialkyl-$C_{1-4}$-aminoalkylgruppe, $C_{1-4}$-Hydroxyalkyl-$C_{1-4}$-aminoalkylgruppe, $C_{1-4}$-Alkoxymethylgruppe, eine Phenylgruppe, die gegebenenfalls mit einem Halogenatom, einer $C_{1-4}$-Alkylgruppe, einer $C_{1-4}$-Alkoxygruppe, einer Nitrogruppe, einer Trifluormethylgruppe, einer Aminogruppe oder einer $C_{1-4}$-Alkylaminogruppe substituiert ist, eine Benzylgruppe, die gegebenenfalls mit einem Halogenatom, einer $C_{1-4}$-Alkylgruppe, einer $C_{1-4}$-Alkoxygruppe, einer Nitrogruppe, einer Trifluormethylgruppe, einer Aminogruppe oder einer $C_{1-4}$-Alkylaminogruppe substituiert ist, einen Heterocyclus, der unter Thiophen, Furan und Pyridin ausgewählt ist, oder auch eine Gruppe $-(CH_2)_p-O-(CH_2)_q-OR''$ worin p und q ganze Zahlen bedeuten, die identisch oder voneinander verschieden sind und im Bereich von 1 bis einschließlich 3 liegen, und R'' Wasserstoff oder Methyl bedeutet.

Die Verbindungen der Formel (II) werden vorzugsweise ausgewählt aus 4,5-Diamino-pyrazol, 4,5-Diamino-1-hydroxyethyl-pyrazol, 1-Benzyl-4,5-diamino-3-methyl-pyrazol, 4,5-Diamino-1-(ß-hydroxyethyl)-3-(4'-methoxyphenyl)-pyrazol, 4,5-Diamino-1-(ß-hydroxyethyl)-3-(4'-methylphenyl)-pyrazol, 4,5-Diamino-1-(ß-hydroxyethyl)-3-(3'-methylphenyl)-pyrazol, 4,5-Diamino-3-methyl-1-isopropyl-pyrazol, 4,5-Diamino-3-(4'-methoxyphenyl)-1-isopropyl-pyrazol, 4,5-Diamino-1-ethyl-3-methylpyrazol, 4,5-Diamino-1-ethyl-3-(4'-methoxyphenyl)-pyrazol, 4,5-Diamino-3-hydroxymethyl-1-methyl-pyrazol, 4,5-Diamino-1-ethyl-3-hydroxymethyl-pyrazol, 4,5-Diamino-3-hydroxymethyl-1-isopropyl-pyrazol, 4,5-Diamino-3-hydroxymethyl-1-tert.-butyl-pyrazol, 4,5-Diamino-3-hydroxymethyl-1-phenyl-pyrazol, 4,5-Diamino-3-hydroxymethyl-1-(2'-methoxyphenyl)-pyrazol, 4,5-Diamino-3-hydroxymethyl-1-(3'-methoxyphenyl)-pyrazol, 4,5-Diamino-3-hydroxymethyl-1-(4'-methoxyphenyl)-pyrazol, 1-Benzyl-4,5-diamino-3-hydroxymethyl-pyrazol, 4,5-Diamino-3-methyl-1-(2'-methoxyphenyl)-pyrazol, 4,5-Diamino-3-methyl-1-(3'-methoxyphenyl)-pyrazol, 4,5-Diamino-3-methyl-1-(4'-methoxyphenyl)-pyrazol, 3-Aminomethyl-4,5-diamino-1-methyl-pyrazol, 3-Aminomethyl-4,5-diamino-1-ethyl-pyrazol, 3-Aminomethyl-4,5-diamino-1-isopropyl-pyrazol, 3-Aminomethyl-4,5-diamino-1-tert.-butyl-pyrazol, 4,5-Diamino-3-dimethylaminomethyl-1-methyl-pyrazol, 4,5-Diamino-3-dimethylaminomethyl-1-ethyl-pyrazol, 4,5-Diamino-3-dimethylaminomethyl-1-isopropyl-pyrazol, 4,5-Diamino-3-dimethylaminomethyl-1-tert.-butyl-pyrazol, 4,5-Diamino-3-ethylaminomethyl-1-methyl-pyrazol, 4,5-Diamino-3-ethylaminomethyl-1-ethyl-pyrazol, 4,5-Diamino-3-ethylaminomethyl-1-isopropyl-pyrazol, 4,5-Diamino-3-ethylaminomethyl-1-tert.-butyl-pyrazol, 4,5-Diamino-3-dimethylaminomethyl-1-methyl-pyrazol, 4,5-Diamino-3-dimethylaminomethyl-1-methyl-pyrazol, 4,5-Diamino-1-ethyl-3-methylaminomethyl-pyrazol, 1-tert.-Butyl-4,5-Diamino-3-methylaminomethyl-pyrazol, 4,5-Diamino-3-[(β-hydroxyethyl)aminomethyl]-1-methyl-pyrazol, 4,5-Diamino-3-[(β-hydroxyethyl)aminomethyl]-1-isopropyl-pyrazol, 4,5-Diamino-1-ethyl-3-[(β-hydroxyethyl)aminomethyl]-pyrazol, 1-tert--Butyl-4,5-diamino-3-[(β-hydroxyethyl)aminomethyl]-pyrazol, 4-Amino-5-(β-hydroxyethyl)-amino-1,3-dimethyl-pyrazol, 4-Amino-5-(β-hydroxyethyl)amino-1-isopropyl-3-methyl-pyrazol, 4-Amino-5-(β-hydroxyethyl)amino-1-ethyl-3-methyl-pyrazol, 4-Amino-5-(β-hydroxyethyl)amino-1-tert.-butyl-3-methyl-pyrazol, 4-Amino-5-(β-hydroxyethyl)amino-1-phenyl-3-methyl-pyrazol, 4-Amino-5-(β-hydroxyethyl)amino-1-(2-methoxyphenyl)-3-methyl-pyrazol, 4-Amino-5-(β-hydroxyethyl)amino-1-(3-methoxyphenyl)-3-methyl-pyrazol, 4-Amino-5-(β-hydroxyethyl)amino-1-(4-methoxyphenyl)-3-methyl-pyrazol, 4-Amino-5-(β-hydroxyethyl)amino-1-benzyl-3 -methyl-pyrazol, 4-Amino-1-ethyl-3-methyl-5-methylamino-pyrazol, 4-Amino-1-tert.-butyl-3-methyl-5-methylamino-pyrazol, 4,5-Diamino-1,3-dimethyl-pyrazol, 4,5-Diamino-3-tert.-butyl-1-methyl-pyrazol, 4,5-Diamino-1-tert.-butyl-3-methyl-pyrazol 4,5-Diamino-1-methyl-3-phenyl-pyrazol, 4,5-Diamino-1-(β-hydroxyethyl)-3-methyl-pyrazol, 4,5-Diamino-1-(β-hydroxyethyl)-3-phenyl-pyrazol, 4,5-Diamino-1-methyl-3-(2'-chlorphenyl)-pyrazol, 4,5-Diamino-1-methyl-3-(4'-chlorphenyl)-pyrazol, 4,5-Diamino-1-methyl-3-(3'-trifluormethylphenyl)-pyrazol, 4,5-Diamino-1,3-diphenyl-pyrazol, 4,5-Diamino-3-methyl-1-phenyl-pyrazol, 4-Amino-1,3-dimethyl-5-phenylamino-pyrazol, 4-Amino-1-ethyl-3-methyl-5-phenylamino-pyrazol, 4-Amino-1,3-dimethyl-5-methylamino-pyrazol, 4-Amino-3-methyl-1-isopropyl-5-methylamino-pyrazol, 4-Amino-3-isobutoxymethyl-1-methyl-5-methylamino-pyrazol, 4-Amino-3-methoxyethoxymethyl-1-methyl-5-methylamino-pyrazol, 4-Amino-3-hydroxymethyl-1-methyl-5-methylamino-pyrazol, 4-Amino-1,3-diphenyl-5-phenylamino-pyrazol, 4-Amino-3-methyl-5-methylamino-1-phenyl-pyrazol, 4-Amino-1,3-dimethyl-5-hydrazino-pyrazol, 5-Amino-3-methyl-4-methylamino-1-phenyl-pyrazol, 5-Amino-1-methyl-4-(N,N-methylphenyl)-amino-3-(4'-chlorphenyl)-pyrazol, 5-Amino-3-ethyl-1-methyl-4-(N,N-methylphenyl)-amino-pyrazol, 5-Amino-1-methyl-4-(N,N-methylphenyl)amino-3-phenyl-pyrazol, 5-Amino-3-ethyl-4-(N,N-methylphenyl)amino-pyrazol, 5-Amino-4-(N,N-methylphenyl)-amino-3-phenyl-pyrazol, 5-Amino-4-(N,N-methylphenyl)amino-3-(4'-methylphenyl)-pyrazol, 5-Amino-3-(4'-chlorphenyl)-4-(N,N-methylphenyl)amino-pyrazol, 5-Amino-3-(4'-methoxyphenyl)-4-(N,N-methylphenyl)amino-pyrazol, 4-Amino-5-methylamino-3-phenyl-pyrazol, 4-Amino-5-ethylamino-3-phenyl-pyrazol, 4-Amino-5-ethylamino-3-(4'-methylphenyl)-pyrazol, 4-Amino-3-phenyl-5-propylamino-pyrazol, 4-Amino-5-butylamino-3-phenyl-pyrazol, 4-Amino-3-phenyl-5-phenylamino-pyrazol, 4-Amino-5-benzylamino-3-phenyl-pyrazol, 4-Amino-5-(4'-chlorphenyl)amino-3-phenyl-pyrazol, 4-Amino-3-(4'-chlorphenyl)-5-phenylamino-pyrazol, 4-Amino-3-(4'-methoxyphenyl)-5-phenylamino-pyrazol, 1-(4'-Chlorbenzyl)-4,5-diamino-3-methyl-pyrazol, 4,5-Diamino-3-hydroxymethyl-1-isopropyl-pyrazol, 4-Amino-1-ethyl-3-methyl-5-methylamino-pyrazol, 4-Amino-5-(2'-amino-ethyl)amino-1,3-dimethyl-pyrazol, 4,5-Diamino-1-(4'-methoxy-benzyl)-pyrazol, 4,5-Diamino-1-(4'-methylbenzyl)-pyrazol, 4,5-Diamino-1-(4'-chlorbenzyl)-pyrazol, 4,5-Diamino-1-(3'-methoxybenzyl)-pyrazol, 4-Amino-1-methyl-5-methylamino-pyrazol, 4-Amino-5-(2'-hydroxyethyl)amino-1-methylpyrazol, 4,5-Diamino-1-ethyl-pyrazol, 4,5-Diamino-1-isopropyl-pyrazol, 4-Amino-1-(2'-hydroxyethyl)-5-(2'-hydroxyethyl)aminopyrazol, 4-Amino-1-(2'-hydroxyethyl)-5-methylamino-pyrazol, 4-Amino-5-methylamino-pyrazol. 4,5-Diaminopyrazol, 4,5-Diamino-1-methyl-pyrazol, 4,5-Diamino-1-benzylpyrazol und 4-Amino-1-methyl-5,5-N,N-dimethylaminpyrazol sowie beliebigen Gemischen der voranstehenden.

Die Verbindungen der Formel (II) werden besonders bevorzugt ausgewählt aus 4,5-Diamino-pyrazol, 4,5-Diamino-1-hydroxyethyl-pyrazol, 4,5-Diamino-1,3-dimethyl-pyrazol, 4,5-Diamino-3-methyl-1-phenyl-pyrazol, 4,5-Diamino-1-methyl-3-phenyl-pyrazol, 4-Amino-1,3-dimethyl-5-hydrazino-pyrazol, 1-Benzyl-4,5-diamino-3-methyl-pyrazol, 4,5-Diamino-3-tert.-butyl-1-methyl-pyrazol, 4,5-Diamino-1-tert.-butyl-3-methyl-pyrazol, 4,5-Diamino-1-(β-hydroxyethyl)-3-methyl-pyrazol, 4,5-Diamino-1-ethyl-3-methyl-pyrazol, 4,5-Diamino-1-ethyl-3-(4'-methoxyphenyl)-pyrazol, 4,5-Diamino-1-ethyl-3-hydroxymethyl-pyrazol, 4,5-Diamino-3-hydroxmethyl-1-methyl-pyrazol, 4,5-Diamino-3-hydroxymethyl-1-isopropyl-pyrazol, 4,5-Diamino-3-methyl-1-isopropyl-pyrazol und 4-Amino-5-(2'-aminoethyl)amino-1,3-dimethyl-pyrazol sowie beliebigen Gemischen der voranstehenden.

Als ganz besonders bevorzugtes Pyrazolderivat wird erfindungsgemäß 4,5-Diamino-1-(2-hydroxyethyl)-pyrazol oder eines seiner physiologisch verträglichen Salze eingesetzt.

Neben den erfindungsgemäßen Verbindungen können die Färbemittel ein oder mehrere weitere Farbstoffvorprodukte enthalten.

Hinsichtlich der in den Färbemitteln einsetzbaren weiteren Farbstoffvorprodukte unterliegt die vorliegende Erfindung keinerlei Einschränkungen.

Das Färbemittel enthält mindestens eine weitere Entwicklerkomponente. Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Amino-pyrazolderivate sowie 2,4,5,6-Tetraaminopyrimidin und dessen Derivate eingesetzt.

Es kann bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1)

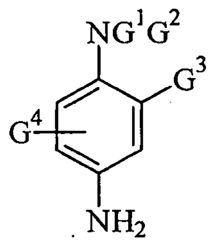

(E1)

wobei

- $G^1$ steht für ein Wasserstoffatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest, einen 4'-Aminophenylrest oder einen $C_1$- bis $C_4$-Alkylrest, der mit einer stickstoffhaltigen Gruppe, einem Phenyl- oder einem 4'-Aminophenylrest substituiert ist;
- $G^2$ steht für ein Wasserstoffatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest oder einen $C_1$- bis $C_4$-Alkylrest, der mit einer stickstoffhaltigen Gruppe substituiert ist;
- $G^3$ steht für ein Wasserstoffatom, ein Halogenatom, wie ein Chlor-, Brom-, Iod- oder Fluoratom, einen $C_1$- bis $C_4$-Alkylrest, einen $C_1$- bis $C_4$-Monohydroxyalkylrest, einen $C_2$- bis $C_4$-Polyhydroxyalkylrest, einen $C_1$- bis $C_4$-Hy-

droxyalkoxyrest, einen $C_1$- bis $C_4$-Acetylaminoalkoxyrest, einen $C_1$- bis $C_4$- Mesylaminoalkoxyrest oder einen $C_1$- bis $C_4$-Carbamoylaminoalkoxyrest;

- $G^4$ steht für ein Wasserstoffatom, ein Halogenatom oder einen $C_1$- bis $C_4$-Alkylrest oder
- wenn $G^3$ und $G^4$ in ortho-Stellung zueinander stehen, können sie gemeinsam eine verbrückende $\alpha,\omega$-Alkylendioxogruppe, wie beispielsweise eine Ethylendioxygruppe bilden.

Beispiele für die als Substituenten in den Verbindungen genannten $C_1$- bis $C_4$-Alkylreste sind die Gruppen Methyl, Ethyl, Propyl, Isopropyl und Butyl. Ethyl und Methyl sind bevorzugte Alkylreste. Bevorzugte $C_1$- bis $C_4$-Alkoxyreste sind beispielsweise eine Methoxy- oder eine Ethoxygruppe. Weiterhin können als bevorzugte Beispiele für eine $C_1$- bis $C_4$-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte $C_2$- bis $C_4$-Polyhydroxyalkylgruppe ist die 1,2-Dihydroxyethylgruppe. Beispiele für Halogenatome sind F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, $C_1$- bis $C_4$-Monoalkylaminogruppen, $C_1$- bis $C_4$-Dialkylaminogruppen, $C_1$- bis $C_4$-Trialkylammoniumgruppen, $C_1$- bis $C_4$-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen verwendet werden können, kann man insbesondere die Verbindungen nennen, die der folgenden Formel (E2) entsprechen, sowie ihre physiologisch verträglichen Salze:

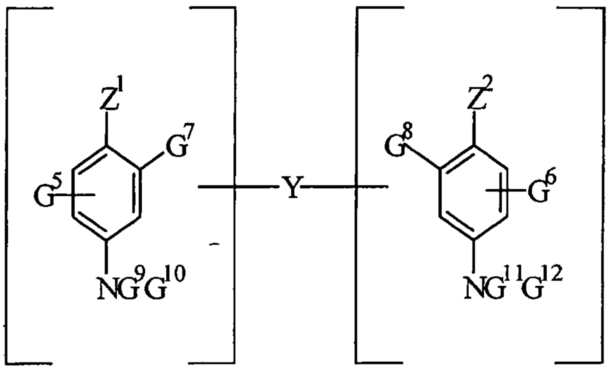

(E2)

wobei:

- $Z^1$ und $Z^2$ stehen unabhängig voneinander für einen Hydroxyl- oder $NH_2$-Rest, der gegebenenfalls durch einen $C_1$- bis $C_4$-Alkylrest, durch einen $C_1$- bis $C_4$-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder $C_1$- bis $C_8$-Alkoxyreste substituiert sein

kann, oder eine direkte Bindung,

- $G^5$ und $G^6$ stehen unabhängig voneinander für ein Wasserstoff- oder Halogenatom, einen $C_1$- bis $C_4$-Alkylrest, einen $C_1$- bis $C_4$-Monohydroxyalkylrest, einen $C_2$- bis $C_4$-Polyhydroxyalkylrest, einen $C_1$- bis $C_4$-Aminoalkylrest oder eine direkte Verbindung zur Verbrückung Y,
- $G^7$, $G^8$, $G^9$, $G^{10}$, $G^{11}$ und $G^{12}$ stehen unabhängig voneinander für ein Wasserstoffatom, eine direkte Bindung zur Verbrückung Y oder einen $C_1$- bis $C_4$-Alkylrest,

mit den Maßgaben, dass

- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3)

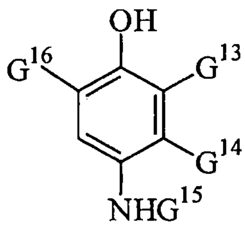

(E3)

wobei:

- $G^{13}$ steht für ein Wasserstoffatom, ein Halogenatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest, einen $C_1$- bis $C_4$-Aminoalkylrest, einen Hydroxy-($C_1$- bis $C_4$)-alkylaminorest, einen $C_1$- bis $C_4$-Hydroxyalkoxyrest, einen $C_1$- bis $C_4$-Hydroxyalkyl-($C_1$-bis $C_4$)-aminoalkylrest oder einen (Di-$C_1$- bis $C_4$-Alkylamino)-($C_1$-bis $C_4$)-alkylrest, und
- $G^{14}$ steht für ein Wasserstoff- oder Halogenatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest, einen $C_1$- bis $C_4$-Aminoalkylrest oder einen $C_1$- bis $C_4$-Cyanoalkylrest,
- $G^{15}$ steht für Wasserstoff, einen $C_1$- bis $C_4$-Alkylrest, einen $C_1$- bis $C_4$-Monohydroxyalkylrest, einen $C_2$- bis $C_4$-Polyhydroxyalkylrest, einen Phenylrest oder einen Benzylrest, und
- $G^{16}$ steht für Wasserstoff oder ein Halogenatom.

Die in Formel (E3) verwendeten Substituenten sind analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die weitere Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die weitere Entwicklerkomponente ausgewählt sein aus heterocyclischen Entwicklerkomponenten, wie beispielsweise den Pyridin-, Pyrimidin-, Pyrazol-PyrimidinDerivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4'-Methoxyphenyl)-amino-3-amino-pyridin, 2,3-Diamino-6-methoxy-pyridin, 2-(β-Methoxyethyl)-amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht:

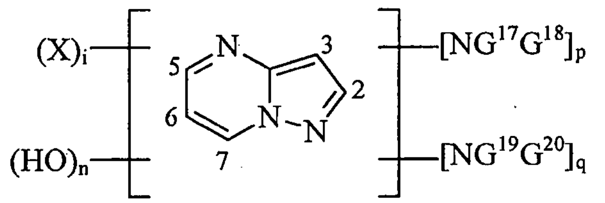

(E4)

wobei:

- $G^{17}$ , $G^{18}$, $G^{19}$ und $G^{20}$ unabhängig voneinander stehen für ein Wasserstoffatom, einen $C_1$- bis $C_4$-Alkylrest, einen Aryl-Rest, einen $C_1$- bis $C_4$-Hydroxyalkylrest, einen $C_2$-bis $C_4$-Polyhydroxyalkylrest einen ($C_1$- bis $C_4$)-Alkoxy-($C_1$- bis $C_4$)-alkylrest, einen $C_1$-bis $C_4$-Aminoalkylrest, der gegebenenfalls durch ein Acetyl-Ureid- oder einen SulfonylRest geschützt sein kann, einen ($C_1$- bis $C_4$)-Alkylamino-($C_1$- bis $C_4$)-alkylrest, einen Di-[($C_1$- bis $C_4$)-alkyl]-($C_1$- bis $C_4$)-aminoalkylrest, wobei die Dialkyl-Reste gegebenenfalls einen Kohlenstoffzyklus oder einen Heterozyklus mit 5 oder 6 Kettengliedern bilden, einen $C_1$- bis $C_4$-Hydroxyalkyl- oder einen Di-($C_1$- bis $C_4$)-[Hydroxyalkyl]-($C_1$- bis $C_4$)-aminoalkylrest,
- die X-Reste stehen unabhängig voneinander für ein Wasserstoffatom, einen $C_1$-bis $C_4$-Alkylrest, einen Aryl-Rest, einen $C_1$- bis $C_4$-Hydroxyalkylrest, einen $C_2$- bis $C_4$-Polyhydroxyalkylrest, einen $C_1$- bis $C_4$-Aminoalkylrest, einen ($C_1$- bis $C_4$)-Alkylamino-($C_1$- bis $C_4$)-alkylrest, einen Di-[($C_1$- bis $C_4$)alkyl]- ($C_1$- bis $C_4$)-aminoalkylrest, wobei die Dialkyl-Reste gegebenenfalls einen Kohlenstoffzyklus oder einen Heterozyklus mit 5 oder 6 Kettengliedern bilden, einen $C_1$- bis $C_4$-Hydroxyalkyl- oder einen Di-($C_1$- bis $C_4$-hydroxyalkyl)-aminoalkylrest, einen Aminorest, einen $C_1$- bis $C_4$-Alkyl- oder Di-($C_1$- bis $C_4$-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,

mit der Maßgabe, dass

- die Summe aus p + q ungleich 0 ist,
- wenn p + q gleich 2 ist, n den Wert 0 hat, und die Gruppen $NG^{17}G^{18}$ und $NG^{19}G^{20}$ belegen die Positionen (2,3); (5,6); (6,7); (3,5) oder (3,7);
- wenn p + q gleich 1 ist, n den Wert 1 hat, und die Gruppen $NG^{17}G^{18}$ (oder $NG^{19}G^{20}$) und die Gruppe OH belegen die Positionen (2,3); (5,6); (6,7); (3,5) oder (3,7);

Die in Formel (E4) verwendeten Substituenten sind analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

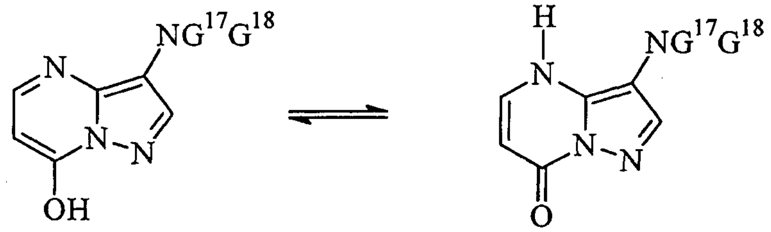

Unter den Pyrazol-[1,5-a]-pyrimidinen der obenstehenden Formel (E4) kann man insbesondere nennen:

- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-Aminopyrazol-[1,5-a]-pyrimidin-7-ol;
- 3-Aminopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1,5-a]-pyrimidin-3-ylamino)-ethanol;
- 2-[(3-Aminopyrazol-[1,5-a]-pyrimidin-7-yl)-(2-hydroxy-ethyl)-amino]-ethanol;
- 2-[(7-Aminopyrazol-[1,5-a]-pyrimidin-3-yl)-(2-hydroxy-ethyl)-amino]-ethanol;
- 5,6-Dimethylpyrazol-[1,5-a]-pyrimidin-3,7-diamin;
- 2,6-Dimethylpyrazol-[1,5-a]-pyrimidin-3,7-diamin;
- 3-Amino-7-dimethylamino-2,5-dimethylpyrazol-[1,5-a]-pyrimidin;

sowie ihre physiologisch verträglichen Salze und ihre tautomeren Formen, wenn ein tautomers Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

Weiterhin können die Färbemittel mindestens eine Kupplerkomponente enthalten.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Bevorzugte Kupplerkomponenten sind

- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,

- Morpholinderivate wie beispielsweise 6-Hydroxybenzomorpholin und 6-Amino-benzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Besonders bevorzugte Kupplerkomponenten sind 1-Naphthol, 1,5-, 2,7-und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Weiterhin können die Färbemittel zur Nuancierung einen oder mehrere direktziehende Farbstoffe enthalten. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäß verwendeten Mittel einen kationischen direktziehenden Farbstoff enthalten. Besonders bevorzugt sind dabei

(a) kationische Triphenylmethanfarbstoffe, wie beispielsweise Basic Blue 7, Basic Blue 26, Basic Violet 2 und Basic Violet 14,

(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie

(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

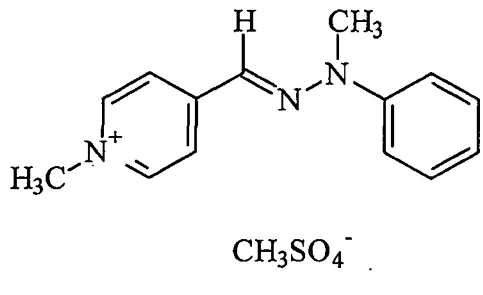

(DZ1)

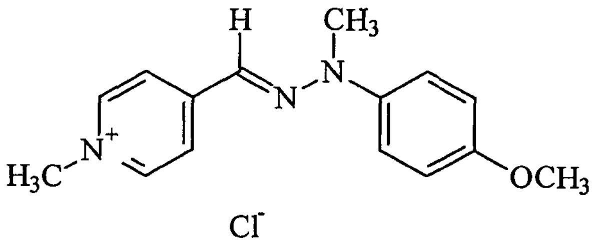

(DZ2)

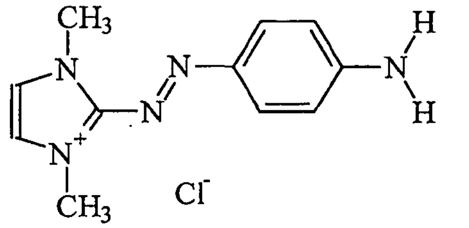

(DZ3)

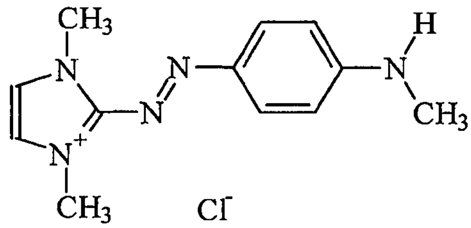

(DZ4)

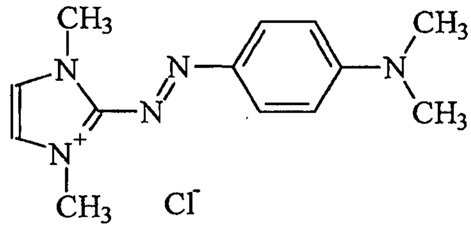

(DZ5)

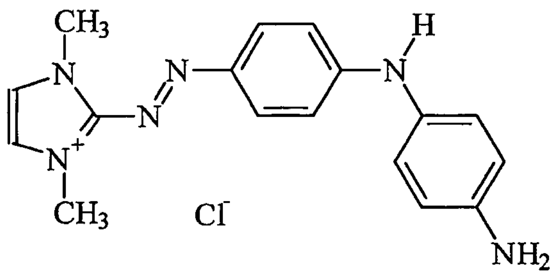

(DZ6)

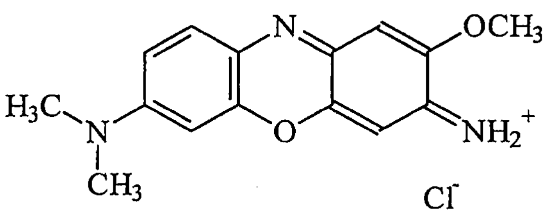

(DZ7)

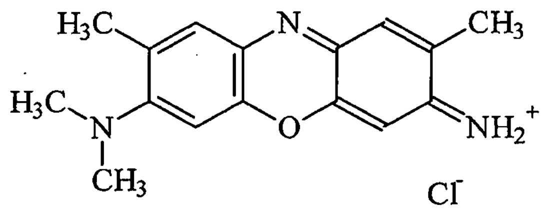

(DZ8)

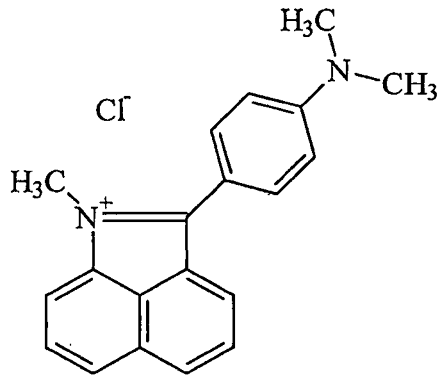

(DZ9)

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor® vertrieben werden, sind ebenfalls ganz besonders bevorzugte kationische direktziehende Farbstoffe.

Die erfindungsgemäß verwendeten Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Im Rahmen der dieser Erfindung zugrundeliegenden Untersuchungen hat sich ferner herausgestellt, dass die erfindungsgemäßen Effekte durch den Einsatz von mindestens einem Silikon und/oder einem Silikongum in den Färbemitteln noch verstärkt werden können. Weiterhin ist daher die erfindungsgemäße Verwendung einer Wirkstoffkombination aus

(a1) 5,6-Dihydroxyindolin oder einem physiologisch verträglichen Salz hiervon und
(b) mindestens einem Silikon und/oder Silikongum

besonders bevorzugt.
Geeignete Silikone oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate.

Beispiele für solche Silikone sind:

- Oligomere Polydimethylcyclosiloxane (INCI-Bezeichnung: Cyclomethicone), insbesondere die tetramere und die

pentamere Verbindung, die als Handelsprodukte DC 344 bzw. DC 345 von Dow Corning vertrieben werden,

- Hexamethyl-Disiloxan (INCI-Bezeichnung: Hexamethyldisiloxane), z. B. das unter der Bezeichnung Abil® K 520 vertriebenen Produkt,
- Polymere Polydimethylsiloxane (INCI-Bezeichnung: Dimethicone), z. B. die unter der Bezeichnung DC 200 von Dow Corning vertriebenen Produkte,
- Polyphenylmethylsiloxane (INCI-Bezeichnung: Phenyl Trimethicone), z. B. das Handelsprodukt DC 556 Fluid von Dow Corning,
- Silicon-Glykol-Copolymere (INCI-Bezeichnung: Dimethicone Copolyol), z. B. die Handelsprodukte DC 190 und DC 193 von Dow Corning,
- Ester sowie Partialester der Silicon-Glykol-Copolymere, wie sie beispielsweise von der Firma Fanning unter der Handelsbezeichnung Fancorsil® LIM (INCI-Bezeichnung: Dimethicone Copolyol Meadowfoamate) vertrieben werden,
- Dimethylsiloxane mit Hydroxy-Endgruppen (INCI-Bezeichnung: Dimethiconol), z. B. die Handelsprodukte DC 1401 und Q2-1403 von Dow Corning,
- aminofunktionelle Polydimethylsiloxane und hydroxylaminomodifizierte Silicone (INCI-Bezeichnung: u. a. Amodimethicone und Quaternium-80), wie die Handelsprodukte XF42-B1989 (Hersteller GE Toshiba Silicones) Q2-7224 (Hersteller: Dow Corning; ein stabilisiertes Trimethylsilylamodimethicon), Dow Corning® 939 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt),
- anionische Silikonöle, wie beispielsweise das Produkt Dow Corning®1784.

Gemäß einer bevorzugten Ausführungsform können die erfingdungsgemäß verwendeten Zubereitungen eine Kombination aus einem flüchtigen und einem nichtflüchtigen Silikon enthalten. Flüchtig im Sinne der Erfindung sind solche Silikone, die ein Flüchtigkeit aufweisen, die gleich oder größer als die Flüchtigkeit des cyclischen, pentameren Dimethylsiloxans ist. Solche Kombinationen sind auch als Handelsprodukte (z. B. Dow Corning®1401, Dow Corning®1403 und Dow Corning®1501, jeweils Mischungen aus einem Cyclomethicone und einem Dimethiconol) erhältlich.

Gemäß einer besonders bevorzugten Ausführungsform wird als Silikon und/oder Silikongum ein Dialkylpolysiloxan oder eines seiner Derivate eingesetzt. Bevorzugt sind die Alkylgruppen Methyl, Ethyl, i-Propyl und n-Propyl. Dimethylpolysiloxan oder eines seiner Derivate wird besonders bevorzugt eingesetzt. Bevorzugte sind die Derivate des Dimethylplysiloxans, die aminofunktionell sind. Ein ganz besonders bevorzugtes Derivat ist unter der INCI-Bezeichnung Amodimethicone im Handel erhältlich.

Die Zubereitungen enthalten die Silikone bevorzugt in Mengen von 0,05 - 10 Gew.-%, insbesondere 0,1 - 5 Gew.-%, bezogen auf die gesamte Anwendungszubereitung.

Weiterhin hat sich im Rahmen der dieser Erfindung zugrundeliegenden Untersuchungen gezeigt, dass spezielle Polymere, welche mindestens eine Monomereinheit der Formel (III)

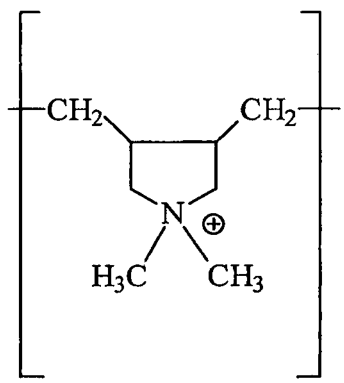

(III)

aufweisen, die erfindungsgemäßen Effekte verstärken können. Die erfindungsgemäße Verwendung einer Wirkstoffkombination aus

(a1) 5,6-Dihydroxyindolin oder einem physiologisch verträglichen Salz hiervon und
(c) einem derartigen Polymeren

ist daher besonders bevorzugt.

In einer ersten bevorzugten Ausführungsform handelt es sich bei den Polymeren mit Monmereinheiten der

Formel (III) um kationische Polymere. Dies sind insbesondere die Homopolymere der Dimethyldiallylammoniumsalze (entspechend den Monomereinheiten der Formel (III)) sowie die Copolymeren der Dimethyldiallylammoniumsalze mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammonium-chlorid-Acrylamid-Copolymer) im Handel erhältlichen Produkte sind Beispiele für solche kationischen Polymere.

In einer zweiten bevorzugten Ausführungsform handelt es sich bei den Polymeren mit Monomereinheiten der Formel (III) um amphotere Polymere. Dies sind insbesondere die Copolymere der Dimethyldiallylammoniumsalze (entspechend den Monomereinheiten der Formel (I)) mit Acrylsäure und/oder Methacrylsäure. Die Copolymeren mit Acrylsäure sind besonders bevorzugt. Das unter dem Handelsnamen Merquat® 280 (Dimethyldiallylammoniumchlorid-Acrylsäure-Copolymer, INCI-Bezeichnung: Polyquaternium-22) vertriebene Produkt ist ein Beispiel für derartige Polymere.

Ferner sind solche amphoteren Copolymere besonders bevorzugt, die neben den Monomereinheiten der Formel (III) und den Acrylsäure- und/oder Methacrylsäureeinheiten weiterhin noch mindestens ein nichtionisches Comonomer enthalten. Beispiele für derartige nichtionische Comonomere sind die Ester oder Amide von Acrylsäure und/oder Methacrylsäure. Das unter der Handelsbezeichnung Merquat® Plus 3330 (Dimethyldiallylammoniumchlorid-Acrylsäure-Acrylamid-Terpolymer, INCI-Bezeichnung: Polyquaternium-39) vertriebene Produkt ist ein Beispiel für ein derartiges bevorzugtes Polymer.

In einer weiteren Ausgestaltungsform der vorliegenden Erfindung ist weiterhin die erfindungsgemäße Verwendung einer Wirkstoffkombination aus

(a1) 5,6-Dihydroxyindolin oder einem physiologisch verträglichen Salz hiervon,
(b) mindestens einem Silikon und/oder Silikongum und
(c) einem Polymeren, welches mindestens eine Monomereinheit der Formel (III) aufweist, bevorzugt.

Die erfindungsgemäß verwendeten Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in den Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,

- lineare Fettsäuren mit 10 bis 22 C-Atomen (Seifen),
- Ethercarbonsäuren der Formel $R\text{-}O\text{-}(CH_2\text{-}CH_2O)_x\text{-}CH_2\text{-}COOH$, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel $R\text{-}O(CH_2\text{-}CH_2O)_x\text{-}SO_3H$, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten $C_8\text{-}C_{22}$-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- $C_{12}$-$C_{22}$-Fettsäuremono- und -diester von Anlagerungsprodukten von 1 bis 30 Mol Ethylenoxid an Glycerin,
- $C_8$-$C_{22}$-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel $R^1O$-$(Z)_x$. Diese Verbindungen sind durch die folgenden Parameter gekennzeichnet.

Der Alkylrest $R^1$ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die verwendbaren Alkylpolyglykoside können beispielsweise nur einen bestimmten Alkylrest $R^1$ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen $R^1$

- im wesentlichen aus $C_8$- und $C_{10}$-Alkylgruppen,
- im wesentlichen aus $C_{12}$- und $C_{14}$-Alkylgruppen,
- im wesentlichen aus $C_8$- bis $C_{16}$-Alkylgruppen oder
- im wesentlichen aus $C_{12}$- bis $C_{16}$-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöle auf dem Haar gewünscht wird, bevorzugt zu dieser Substanzklasse als weiterem Inhaltsstoff der Zubereitungen zurückgreifen.

Auch die alkoxylierten Homologen der genannten Alkylpolyglykoside können eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine $-COO^{(-)}$- oder $-SO_3^{(-)}$-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer $C_8$-$C_{18}$-Alkyl- oder Acylgruppe im Molekül mindestens eine freie Aminogruppe und mindestens eine -COOH- oder $-SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylamino-

ethylaminopropionat und das $C_{12-18}$-Acylsarcosin.

Als kationische Tenside werden insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxy-ethyl)dimethylammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine besonders geeignete Verbindung aus dieser Substanzgruppe stellt das unter der Bezeichnung Tegoamid® S 18 im Handel erhältliche Stearamidopropyl-dimethylamin dar.

Weitere verwendbare kationische Tenside stellen die quaternisierten Proteinhydrolysate dar.

Ein Beispiel für ein als kationisches Tensid einsetzbares quaternäres Zuckerderivat stellt das Handelsprodukt Glucquat®100 dar, gemäß INCI-Nomenklatur ein "Lauryl Methyl Gluceth-10 Hydroxypropyl Dimonium Chloride".

Weiterhin bevorzugte kationische Tenside, die auch pflegende Eigenschaften aufweisen, sind die Verbindungen der Formel (IV)

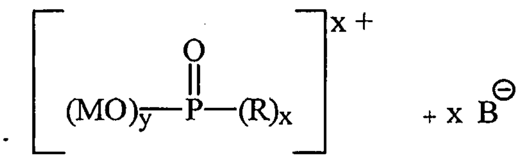
(IV)

In der Formel (IV) steht y für eine ganze Zahl von 0 bis 2, x für eine ganze Zahl von 1 bis 3 mit der Maßgabe, daß die Summe aus x und y gleich 3 ist.

In den Verbindungen der Formel (IV) steht ferner M für Wasserstoff, ein Äquivalent eines Alkali- oder Erdalkalimetallkations, ein Ammoniumkation oder einen Alkylrest mit 1 bis 4 Kohlenstoffatomen, der gegebenenfalls mit einer oder mehreren Hydroxygruppe(n) substituiert ist. Besonders bevorzugt sind Verbindungen, bei denen M für ein Natriumkation steht.

Weiterhin steht B in der Formel (IV) der einzusetzenden Tenside für ein Äquivalent eines physiologisch verträglichen Anions. Als Anion eignen sich beispielsweise Chlorid, Bromid, Jodid, Sulfat, Perchlorat, Tetrafluorborat, Tetraphenylborat und Tetrachlorozinkat. Bevorzugt ist das Chloridion.

R steht in den Tensiden der Formel (IV) für einen Rest der Formel (V),

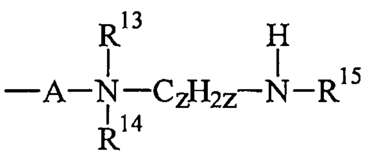
(V)

in der z für eine ganze Zahl von 1 bis 4, insbesondere für 3, steht und $R^{13}$ und $R^{14}$ unabhängig voneinander für einen $C_1$- bis $C_4$-Alkylrest stehen, der gegebenenfalls mit einer oder mehreren Hydroxygruppe(n) oder einer Acylgruppe substituiert ist.

A steht für eine der Einheiten $-O-CH_2-CH_2-CH_2-$, $-O-CH_2-CH_2-$ oder $-O-CH_2-CHOH-CH_2-$, wobei die Einheit $-O-CH_2-CHOH-CH_2-$ besonders bevorzugt ist.

Der Rest $R^{15}$ steht für

(a) einen verzweigten oder unverzweigten, gesättigten $C_8$- bis $C_{18}$-Acylrest oder
(b) einen verzweigten oder unverzweigten, einfach oder mehrfach ungesättigten $C_8$- bis $C_{18}$-Acylrest.

Besonders bevorzugte gesättigte Reste $R^{15}$ sind der Rest der Stearinsäure sowie die Reste der Mischung der Fettsäuren, die man aus Kokosöl gewinnt.

Ein besonders bevorzugter ungesättigter Rest $R^{15}$ ist der Rest der Linolsäure. Überraschenderweise wurde gefunden, dass sich Verbindungen der Formel (IV), bei denen $R^{15}$ der Rest der Linolsäure ist, durch eine höhere Verträglichkeit mit Emulgatorsystemen auszeichnen. Dies bedeutet, dass sich diese Substanzen leichter in die Formulierungen einarbeiten lassen. Weiterhin weisen Formulierungen mit Verbindungen der Formel (IV), bei denen $R^{15}$ für den Rest der Linolsäure steht, einen deutlich höheren Pflegeeffekt im Vergleich zu Verbindungen mit gesättigten Fettsäureresten auf.

Ganz besonders bevorzugte Verbindungen der Formel (IV) sind die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA®, Phospholipid PTC® sowie Phospholipid SV® vertrieben.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Ferner können die erfindungsgemäßen verwendeten Färbemittel weitere Wirk-, Hilfs- und Zusatzstoffe, wie beispielsweise

- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, mit Diethylsulfat quaternierte Dimethylamino-ethylmethacrylat-Vinylpyrrolidon-Copolymere, Vinylpyrrolidon-Imidazolinium-methochlorid-Copolymere und quaternierter Polyvinylalkohol,
- zwitterionische und amphotere Polymere wie beispielsweise Acrylamidopropyl-trimethylammoniumchlorid/Acrylat-Copolymere. und Octylacrylamid/Methyl-methacrylat/tert-Butylaminoethylmethacrylab2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, VinylacetatlButylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Soja-protein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,

- Vitamine, Provitamine und Vitaminvorstufen, insbesondere solche der Gruppen A, $B_3$, $B_5$, $B_6$, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Pölyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, $N_2O$, Dimethylether, $CO_2$ und Luft,
- Antioxidantien,

enthalten.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die erfindungsgemäß verwendeten Mittel enthalten die Farbstoffvorprodukte bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, die Farbstoffvorprodukte in eine pulverförmige oder auch Tabletten-formige Formulierung zu integrieren.

Unter wässrig-alkoholischen Lösungen sind im Sinne der vorliegenden Erfindung wässrige Lösungen enthaltend 3 bis 70 Gew.-% eines $C_1$-$C_4$-Alkohols, insbesondere Ethanol bzw. Isopropanol, zu verstehen. Die erfindungsgemäß verwendeten (Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Die eigentliche oxidative Färbung der Fasern kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Das Oxidationsfärbemittel kann aber auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, Iodide, Chinone oder bestimmte Enzyme.

Geeignete Metallionen sind beispielsweise $Zn^{2+}$, $Cu^{2+}$, $Fe^{2+}$, $Fe^{3+}$, $Mn^{2+}$, $Mn^{4+}$, $Li^{+}$, $Mg^{2+}$, $Ca^{2+}$ und $Al^{3+}$. Besonders geeignet sind dabei $Zn^{2+}$, $Cu^{2+}$ und $Mn^{2+}$. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.

- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,

- Tyrosinase-Oxidase und Tyrosin,
- Uricase und Harnsäure oder deren Salze,
- Cholinoxidase und Cholin,
- Aminosäure-Oxidase und Aminosäuren.

Das eigentliche Haarfärbemittel wird zweckmäßigerweise unmittelbar vor der Anwendung durch Mischung der Zubereitung des Oxidationsmittels mit der Zubereitung, enthaltend die Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann die Zubereitung mit den Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

**Ausführungsbeispiele**

Die folgenden Angaben verstehen sich, soweit nichts anderes vermerkt ist, in Gewichtsprozent.

Es wurden die folgenden Färbecremes hergestellt:

Färbecreme A

| | |
|---|---|
| Cetylalkohol | 14,00 |
| Glyzerinmonostearat NSE[1] | 2,10 |
| Cetiol® V[2] | 2,50 |
| Plantaren® 2000[3] | 0,50 |
| Hycrylmulgol® 012[4] | 5,45 |
| Kaliumoleinseife (12,5%ig) | 10,00 |
| Ammoniumrohagit-Lösung (6%ig)[5] | 5,00 |
| Ammoniumcarbopol-Lösung (1%ig)[6] | 10,00 |
| Titandioxid | 0,50 |
| Tetrasodium EDTA | 0,20 |
| Ammoniak (25%ig) | 8,00 |
| Ascorbinsäure | 0,50 |
| Natriumsulfit | 0,10 |
| Phospholipid® EFA[7] | 1,00 |
| Mirapol® A15[8] | 0,25 |
| Parfüm | 0,4 |
| 5,6-Dihydroxyindolin-Hydrobromid | 0,02 |
| 2-Methylresorcin | 0,10 |
| p-Toluylendiamin-sulfat | 1,45 |
| Resorcin | 0,50 |
| 2-Amino-3-hydroxypyridin | 0,09 |
| m-Aminophenol | 0,12 |
| 3-Metyl-4-aminophenol | 0,01 |
| 2,2'-Dihydroxy-4,4'-diamino-diphenylmethan-dihydrochlorid | 0,08 |
| 4-(2-Hydroxyethyl)amino-2-aminoanisol | 0,03 |
| 2-Chlor-3-amino-6-methylphenol | 0,02 |

(fortgesetzt)

| | |
|---|---|
| Wasser | ad 100 |

[1] (INCI-Bezeichnung: Glyceryl Strearate) (Oleofina)
[2] Ölsäuredecylester (INCI-Bezeichnung: Decyl Oleate) (Cognis)
[3] $C_{8-16}$-Alkyl-1,4-Polyglucosid (ca. 51% Aktivsubstanz; INCI-Bezeichnung: Decyl Glucoside) (Cognis)
[4] Oleylalkohol mit 10 EO-Einheiten (Chemische Fabrik Dr. Angele GmbH)
[5] Lösung eines Ammoniumsalzes eines Acrylsäure-Polymeren (INCI-Bezeichnung: Ammonium Acrylates Copolymer) (Goodrich)
[6] Lösung eines Ammoniumsalzes eines Methacrylsäure-Methylacrylat-Copolymeren (INCI-Bezeichnung: Ammonium Polyacrylate) (Röhm GmbH)
[7] ca. 30% Aktivsubstanz; (INCI-Bezeichnung: Linoleamidopropyl PG-Dimonium Chloride Phosphate) (Mona)
[8] ca. 64% Aktivsubstanz (INCI-Bezeichnung: Polyquaternium-2) (Rhodia)

Färbecreme B

| | |
|---|---|
| Ammoniumcarbopollösung (1%ig) | 14,00 |
| Lanette® E[9] | 0,8 |
| Natriumlaurylethersulfat (27%ige wässrige Lösung) | 4,50 |
| PEG 400 | 0,60 |
| Kaliumoleat (12%ige wässrige Lösung) | 3,50 |
| Titandioxid | 0,40 |
| Cetylstearylalkohol 50/50 | 10,00 |
| Eumulgin® B2[10] | 3,00 |
| Eutanol@ G[11] | 2,00 |
| Cutina® AGS [12] | 2,00 |
| Cutina® GMS-SE[13] | 1.5ß |
| Amodimethicone XF 42-B1989[14] | 1,45 |
| Kalilauge | 0,50 |
| Tetrasodium EDTA | 0,20 |
| Natriumsulfit | 0,10 |
| Ascorbinsäure | 0,10 |
| Merquat® Plus 3330[15] | 2,00 |
| Parfüm | 0,50 |
| Ammoniak25%ig | 7,00 |
| Aerosil® 200[16] | 0,25 |
| Phospholipid® EFA | 0,10 |
| 5,6-Dihydroxyindolin-Hydrobromid | 0,03 |
| Methylresorcin | 0,10 |
| p-Toluylendiamin-sulfat | 1,70 |
| Resorcin | 0,70 |
| 2-Amino-3-hydroxypyridin | 0,15 |
| m-Aminophenol | 0,10 |
| 3-Metyl-4-aminophenol | 0,01 |
| 2,2'-Dihydroxy-4,4'-diamino-diphenylmethan-dihydrochlorid | 0,09 |
| 1-(2-Hydroxyethyl)-4,5-diamino-pyrazol-sulfat | 0,20 |
| 4-Amino-2-hydroxytoluol | 0,10 |
| 4-(2-Hydroxyethyl)amino-2-aminoanisol | 0,04 |

(fortgesetzt)

| | |
|---|---|
| Wasser | ad. 100 |

[9] Cetylstearylalkoholsulfat-Natrium-Salz (INCI-Bezeichnung: Sodium Cetearyl Sulfate) (Cognis)
[10] Cetylstearylalkohol mit ca. 20-EO-Einheiten (INCI-Bezeichnung: Ceteareth-20) (Cognis)
[11] 2-Octyldodecylalkohol (INCI-Bezeichnung: Octyldodecanol) (Cognis)
[12] Ethylenglykoldistearat (INCI-Bezeichnung: Glycol Distearate) (Cognis)
[13] Glycerinmono-distearat-Kaliumstearat-Gemisch auf pflanzlicher Basis (INCI-Bezeichnung: Glyceryl Stearate SE) (Cognis)
[14] aminofunktionelles Silicon (INCI-Bezeichnung: Amodimethicone) (GE-Toshiba-Silicones)
[15] Dimethyldiallylammoniumchlorid-Acrylsäure-Acrylamid-Terpolymer (ca. 9,5% Aktivsubstanzgehalt; INCI-Bezeichnung: Polyquaternium-39) (Nalco)
[16] pyrogene Kieselsäure (INCI-Bezeichnung: Silica) (Degussa)

Färbecreme C

| | |
|---|---|
| Cetylalkohol | 16,00 |
| Glyzerinmonostearat NSE | 2,50 |
| Cetiol® V | 2,00 |
| Plantaren® 2000 | 0,50 |
| Hycrylmulgol® 012 | 5,00 |
| Kaliumoleinseife (12,5%ig) | 12,00 |
| Ammoniumrohagit-Lösung (6%ig) | 5,00 |
| Ammoniumcarbopol-Lösung (1%ig) | 10,00 |
| Amodimethicone XF 42-B1989® | 1,45 |
| Titandioxid | 0,50 |
| Tetrasodium EDTA | 0,20 |
| Ammoniak (25%ig) | 8,00 |
| Ascorbinsäure | 0,50 |
| Natriumsulfit | 0,10 |
| Phospholipid® EFA | 1,00 |
| Mirapol® A15 | 0,25 |
| Parfüm | 0,4 |
| 5,6-Dihydroxyindolin-Hydrobromid | 0,10 |
| 2-Methylresorcin | 0,15 |
| p-Toluylendiamin-sulfat | 1,05 |
| Resorcin | 0,30 |
| 2-Amino-3-hydroxypyridin | 0,07 |
| m-Aminophenol | 0,12 |
| 3-Metyl-4-aminophenol | 0,01 |
| 2,4,5,6-Tetraaminopyrimidin | 0,20 |
| Wasser | ad. 100 |

Als Vergleichsrezepturen wurden die Färbecremes A', B' und C' hergestellt, die im wesentlichen den Färbecremes A, B und C entsprechen, jedoch kein 5,6-Dihydroxyindolin-hydrobromid enthalten.

Ferner wurde die folgende Oxidationsmittelzubereitung hergestellt:

| | |
|---|---|
| Emulgade® F[17] | 2,5 |
| o-Phosphorsäure | 0,05 |
| Natriumbenzoat | 0,05 |
| Wasserstoffperoxid (35%ig in Wasser) | 17,00 |

(fortgesetzt)

| | |
|---|---|
| Disodium-EDTA | 0,2 |
| Natriumpyrophosphat | 0,3 |
| Wasser | ad 100 |
| pH-Wert | 3,0 - 3,4 |

[17] Cetylstearylalkohol-Emulgatoren-Gemisch (INCI-Bezeichnung: Cetearyl Alcohol, PEG-40 Castor Oil, Sodium Cetearyl Sulfate) (Cognis)

Unmittelbar vor der Anwendung wurden die Färbecremes A, B und C sowie A', B' und C' jeweils mit der Oxidationsmittelzubereitung im Verhältnis 1:1 vermischt. Die resultierenden Anwendungszubereitungen wurden im Halbseitenversuch entsprechend der folgenden Abbildung auf die Haare von Probanden aufgetragen. Nach 30 Minuten Einwirkzeit wurden die Haare gründlich mit Wasser gespült und anschließend getrocknet.

*Abbildung:*

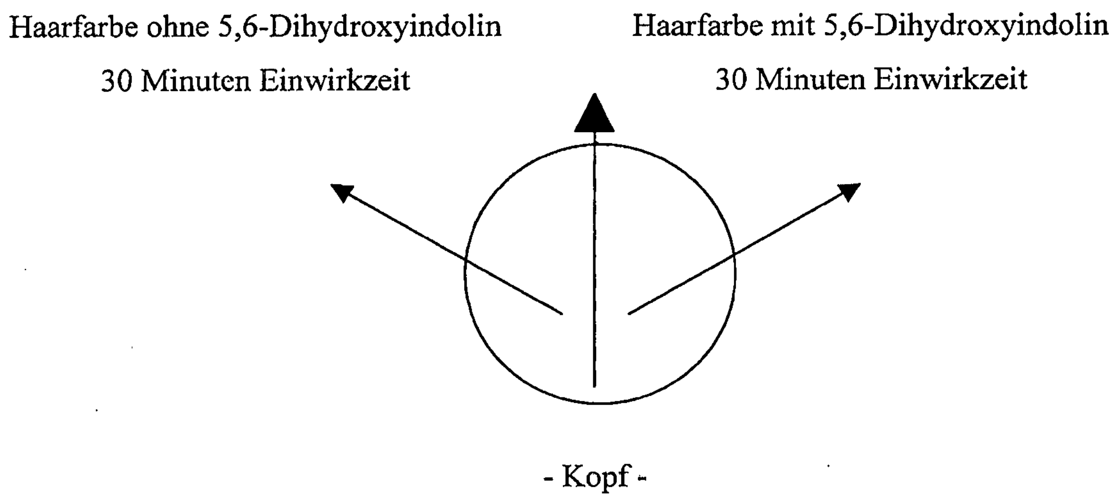


Bei diesem Versuch wurde festgestellt, dass der farbliche Ausgleich vom frisch nachgewachsenen Ansatz bis zu den bereits diversen Einflüssen ausgesetzten Haarlängen bei einer Zugabe 5,6-Dihydroxyindolin besser war (siehe Tabelle).

Die $\Delta$E-Werte des CIELAB-Farbsystems wurden gemäß Gleichung (I) ermittelt:

$$\Delta E = \sqrt{(\Delta L)^2 + (\Delta A)^2 + (\Delta B)^2} \quad (I)$$

*Tabelle:*

| Nuance | Naturhaar | Farbergebnis | LAB-Wert Ansatz | LAB-Wert Länge | ΔE-Werte |
|---|---|---|---|---|---|
| Färbecreme A' (ohne 5,6-Dihydroxyindolin) | Hellbraun Cendre Ergrauung: 0% | Ansatz: Mittelbraun Schoko Längen: Mittelbraun Gold | L: 22,31 A: 3,17 B: 3,34 | L: 23,12 A: 2,49 B: 3,62 | 1,09 |

(fortgesetzt)

| Nuance | Naturhaar | Farbergebnis | LAB-Wert Ansatz | LAB-Wert Länge | ΔE-Werte |
|---|---|---|---|---|---|
| Färbecreme A<br>(mit 0,1 % 5,6-Dihydroxyindolin) | Hellbraun Cendre<br>Ergrauung: 0% | Ansatz: Mittelbraun Schoko<br>Längen: Mittelbraun Schoko (gleichmäßiges Ergebnis) | L: 22,31<br>A: 3,17<br>B: 3,34 | L: 22,35<br>A: 3,16<br>B: 3,36 | 0,05 |
| Färbecreme B'<br>(ohne 5,6-Dihydroxyindolin) | Hellbraun<br>Ergrauung: 80% | Ansatz: Hellbraun Schoko Rot<br>Längen: DunkelblondSchoko Rot | L: 24,02<br>A: 6,37<br>B: 5,79 | L: 25,75<br>A: 8,13<br>B: 8,09 | 3,37 |
| Färbecreme B<br>(mit 0,1 % 5,6-Dihydroxyindolin) | Hellbraun<br>Ergrauung: 80% | Ansatz: Hellbraun Schoko<br>Längen: Hellbraun Schoko (gleichmäßiges Ergebnis)<br>Sehr gute Grauabdeckung | L: 23,74<br>A: 6,03<br>B: 5,13 | L: 23,82<br>A: 6,07<br>B: 5,13 | 0,09 |
| Färbecreme C'<br>(ohne 5,6-Dihydroxyindolin) | Dunkelblond<br>Ergrauung: 50% | Ansatz: Dunkelblond Schoko<br>Längen: Dunkelblond Schoko Rot | L: 26,69<br>A: 8,99<br>B: 9,97 | L: 25,75<br>A: 8,13<br>B: 8,09 | 2,27 |
| Färbecreme C<br>(mit 0,1 % 5,6-Dihydroxyindolin) | Dunkelblond<br>Ergrauung: 50% | Ansatz: Dunkelblond Schoko<br>Längen: Dunkelblond Schoko (gleichmäßiges Ergebnis) | L: 26,69<br>A: 8,99<br>B: 9,97 | L: 26,57<br>A: 8,86<br>B: 10,01 | 0,18 |

**Patentansprüche**

1. Verwendung von 5.6-Dihydroxyindolin oder eines physiologisch verträglicheen Salzes davon zur Verbesserung des Egalisierungsvermögens von Färbemitteln für keratinische Fasern, die mindestens eine Entwicklerkomponente und gegebenenfalls mindestens eine Kupplerkomponente enthalten, **dadurch gekennzeichnet, dass** das 5,6-Dihydroxyindolin oder ein physiologisch verträgliches Salz davon gemeinsam mit den übrigen Farbstoffvorprodukten auf die Fasern aufgetragen werden.

2. Verwendung nach Anspruch 1, **dadurch gekennzeichnet, dass** das Färbemittel mindestens ein 4,5-Diaminopyrazolderivat enthalt.

3. Verwendung nach Anspruch 2, **dadurch gekennzeichnet, dass** das Färbemittel mindestens ein 4,5-Diaminopyrazolderivat der Formel (II) enthält

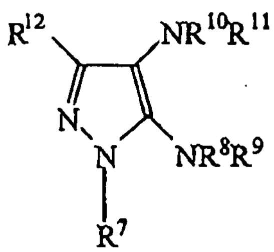

(II),

worin
$R^7$, $R^8$, $R^9$, $R^{10}$ und R" unabhängig voneinander stehen für Wasserstoff, eine geradkettige oder verzweigte $C_{1-6}$-Alkylgruppe, eine $C_{2-4}$-Hydroxyalkylgruppe, eine $C_{2-4}$-Aminoalkylgruppe, eine Phenylgruppe, die gegebenenfalls mit einer Nitrogruppe, einer Trifluormethylgruppe, einer Aminogruppe oder einer $C_{1-4}$-Alkylaminogruppe substituiert ist, eine Benzylgruppe, die gegebenenfalls mit einem Halogenatom, einer $C_{1-4}$-Alkylgruppe, einer $C_{1-4}$-Alkoxygruppe, einer Methylendioxygruppe oder einer Aminogruppe substituiert ist, eine Pyridylgruppe, eine Thienylgruppe, eine

Furylgruppe oder eine Gruppe

$$-(CH_2)_m-P-(CH)_n-Z$$

|

Q ,

worin m und n ganze Zahlen bedeuten, die identisch oder voneinander verschieden sind und im Bereich von 1 bis einschließlich 3 liegen, P steht für Sauerstoff oder eine Gruppe NH, Q steht für Wasserstoff oder Methyl und Z steht für Methyl, eine Gruppe OR oder NRR', worin R und R', die identisch oder voneinander verschieden sein können, Wasserstoff, Methyl oder Ethyl bedeuten,
wobei wenn $R^a$ Wasserstoff bedeutet, $R^9$ auch $C_{1-4}$-Alkylamino bedeuten kann, und $R^{12}$ steht für eine geradkettige oder verzweigte $C_{1-6}$-Alkylgruppe, $C_{1-4}$-Hydroxyalkylgruppe, $C_{1-4}$-Aminoalkylgruppe, $C_{1-4}$-Alkyl-$C_{1-4}$-aminoalkylgruppe, $C_{1-4}$-Dlalkyl-$C_{1-4}$-aminoalkylgruppe, $C_{1-4}$-Hydroxyalkyl-$C_{1-4}$-aminoalkylgruppe, $C_{1-4}$-Alkoxymethylgruppe, eine Phenylgruppe, die gegebenenfalls mit einem Halogenatom, einer $C_{1-4}$-Alkylgruppe, einer $C_{1-4}$-Alkoxygruppe, einer Nitrogruppe, einer Trifluormethylgruppe, einer Aminogruppe oder einer $C_{1-4}$-Alkylaminogruppe substituiert ist, eine Benzylgruppe, die gegebenenfalls mit einem Halogenatom, einer $C_{1-4}$-Alkylgruppe, einer $C_{1-4}$-Alkoxygruppe, einer Nitrogruppe, einer Trifluormethylgruppe, einer Aminogruppe oder einer $C_{1-4}$-Alkylaminogruppe substituiert ist, einen Heterocyclus, der unter Thiophen, Furan und Pyridin ausgewählt ist, oder auch eine Gruppe $-(CH_2)_p-O-(CH_2)_q-OR''$ worin p und q ganze Zahlen bedeuten, die identisch oder voneinander verschieden sind und im Bereich von 1 bis einschließlich 3 liegen, und R'' Wasserstoff oder Methyl bedeutet.

4. Verwendung nach Anspruch 3, **dadurch gekennzeichnet, dass** als 4,5-Diaminopyrazolderivat 4,5-Diamino-1-(2-hydroxyethyl)-pyrazol oder eines seiner physiologisch verträglichen Salze eingesetzt wird.

5. Verwendung nach einem der Ansprüche 1 bis 4, **dadurch gekennzeichnet, dass** das Färbemittel weiterhin mindestens ein Silikon und/oder ein Silikongum enthält.

6. Verwendung nach einem der Ansprüche 1 bis 5, **dadurch gekennzeichnet, dass** das Färbemittel mindestens ein Polymer, enthaltend mindestens eine Monomereinheit der Formel (III)

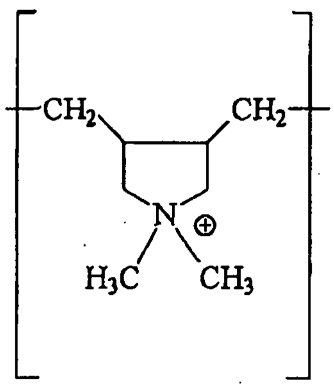

(III) .

enthält.

## Claims

1. Use of 5,6-dihydroxyindoline or a physiologically acceptable salt thereof for improving the leveling power of dyes for keratinic fibers, the dyes containing at least one developer component and optionally at least one coupler component, **characterized in that** 5,6-dihydroxyindoline or a physiologically acceptable salt thereof together with the customary dye precursors are applied to the fibers.

2. Use according to Claim 1, **characterized in that** the dye contains at least one 4,5-diaminopyrazole derivative.

3. Use according to Claim 2, **characterized in that** the dye contains at least one 4,5-diaminopyrazole derivative of formula (II)

(II), 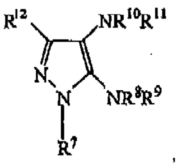 ,

where
$R^7$, $R^8$, $R^9$, $R^{10}$, and $R^{11}$ independently stand for hydrogen, a straight-chain or branched $C_{1-6}$ alkyl group, a $C_{2-4}$ hydroxyalkyl group, a $C_{2-4}$ aminoalkyl group, a phenyl group which is optionally substituted with a nitro group, a trifluoromethyl group, an amino group, or a $C_{1-4}$ alkylamino group, a benzyl group which is optionally substituted with a halogen atom, a $C_{1-4}$ alkyl group, a $C_{1-4}$ alkoxy group, a methylenedioxy group, or an amino group, a pyridyl group, a thienyl group, a furyl group, or a group

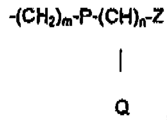 ,

where m and n stand for integers which are identical or different and are in the range from 1 to and including 3, P stands for oxygen or an NH group, Q stands for hydrogen or methyl, and Z stands for methyl or an OR or NRR' group, where R and R', which may be identical or different, stand for hydrogen, methyl, or ethyl,
wherein when $R^6$ stands for hydrogen, $R^9$ may also stand for $C_{1-4}$ alkylamino, and $R^{12}$ stands for a straight-chain or branched $C_{1-6}$ alkyl group, $C_{1-4}$ hydroxyalkyl group, $C_{1-4}$ alkylamino group, $C_{1-4}$ alkyl-$C_{1-4}$ aminoalkyl group, $C_{1-4}$ dialkyl-$C_{1-4}$ aminoalkyl group, $C_{1-4}$ hydroxyalkyl-$C_{1-4}$ aminoalkyl group, or $C_{1-4}$ alkoxymethyl group, a phenyl group which is optionally substituted with a halogen atom, a $C_{1-4}$ alkyl group, a $C_{1-4}$ alkoxy group, a nitro group, a trifluoromethyl group, an amino group, or a $C_{1-4}$ alkylamino group, a benzyl group which is optionally substituted with a halogen atom, a $C_{1-4}$ alkyl group, a $C_{1-4}$ alkoxy group, a nitro group, a trifluoromethyl group, an amino group, or a $C_{1-4}$ alkylamino group, a heterocycle which is selected from thiophene, furan, and pyridine, or a group $-(CH_2)_p-O-(CH_2)_c-OR''$, where p and q stand for integers which are identical or different and are in the range from 1 to and including 3, and R'' stands for hydrogen or methyl.

4. Use according to Claim 3, **characterized in that** 4,5-diamino-1-(2-hydroxyethyl)pyrazole or one of its physiologically acceptable salts is used as the 4,5-diaminopyrazole derivative.

5. Use according to one of Claims 1 through 4, **characterized in that** the dye also contains at least one silicone and/or one silicone gum.

6. Use according to one of Claims 1 through 5, **characterized in that** the dye contains at least one polymer which contains at least one monomer unit of formula (III)

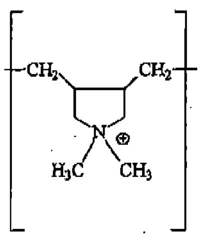 (III)

## Revendications

1. Utilisation de 5,6-dihydroxyindoline ou d'un de ses sels physiologiquement compatibles pour améliorer la capacité d'égalisation de colorants pour des fibres de kératine contenant au moins un composant de développement et, éventuellement, au moins un composant de couplage, **caractérisée en ce que** la 5,6-dihydroxyindoline ou un de ses sels physiologiquement compatibles est appliquée sur la fibre en association avec les produits colorants traditionnels.

2. Utilisation selon la revendication 1, **caractérisée en ce que** le colorant contient au moins un dérivé du 4,5-diaminopyrazole.

3. Utilisation selon la revendication 2, **caractérisée en ce que** le colorant contient au moins un dérivé du 4,5-diaminopyrazole de formule (II) :

(II). 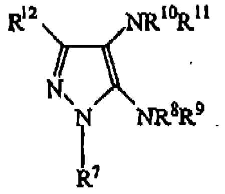

Où $R^7$, $R^8$, $R^9$, $R^{10}$ et $R^{11}$ représentent indépendamment hydrogène, un groupe alkyle linéaire ou ramifié $C_{1-6}$, un groupe hydroxyalkyle $C_{2-4}$, un groupe aminoalkyle $C_{2-4}$, un groupe phényle éventuellement substitué par un groupe nitro, trifluorotriméthyle, amino ou alkylamino $C_{1-4}$, un groupe benzyle éventuellement substitué par un atome d'halogène, un groupe alkyle $C_{1-4}$, alcoxy $C_{1-4}$, méthylènedioxy ou amino, un groupe pyridyle, un groupe thiényle, un groupe furyle ou un groupe

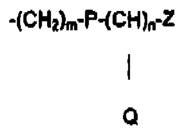

où m et n sont des entiers identiques ou différents l'un de l'autre et compris entre 1 et 3 inclus, P est un atome d'oxygène ou un groupe NH, Q est un atome d'hydrogène ou un groupe méthyle, et Z représente un groupe méthyle, OR ou NRR', où R et R', identiques ou différents, peuvent être un hydrogène, méthyle ou éthyle,
où si $R^8$ est un hydrogène, $R^9$ peut aussi représenter un alkylamino $C_{1-4}$, et $R^{12}$ représente un groupe alkyle linéaire ou ramifié $C_{1-6}$, un groupe hydroxyalkyle $C_{1-4}$, aminoalkyle $C_{1-4}$, alkyl-$C_{1-4}$-aminoalkyle, $C_{1-4}$, dialkyl-$C_{1-4}$-aminoalkyle $C_{1-4}$, hydroxyalkyl-$C_{1-4}$-aMinoalkyle $C_{1-4}$, alcoxyalkyle $C_{1-4}$, un groupe phényle éventuellement substitué par un atome d'halogène, un groupe alkyle $C_{1-4}$, alcoxyalkyle $C_{1-4}$, nitro, trifluorométhyle, amino ou alkylamino $C_{1-4}$, un groupe benzyle éventuellement substitué par un atome d'halogène, un groupe alkyle $C_{1-4}$, alcoxyalkyle $C_{1-4}$, nitro, trifluorométhyle, amino ou alkylamino $C_{1-4}$, un hétérocycle choisi parmi thiophène, furane et pyridine, ou encore un groupe $-(CH_2)_p-O-(CH_2)_q-OR''$, où p et q sont des entiers identiques ou différents l'un de l'autre et compris entre 1 et 3 inclus, et où R'' représente un atome d'hydrogène ou un groupe méthyle.

4. Utilisation selon la revendication 3, **caractérisée en ce qu'**on utilise comme dérivé du 4,5-diaminopyrazole le 4,5-diamino-1-(2-hydroxyéthyl)-pyrazole ou un de ses sels physiologiquement compatibles.

5. Utilisation selon l'une quelconque des revendications 1 à 4, **caractérisée en ce que** le colorant comprend en outre au moins une silicone ou une gomme silicone.

6. Utilisation selon l'une quelconque des revendications 1 à 5, **caractérisée en ce que** le colorant contient au moins un polymère contenant au moins une unité monomère de formule (III) :

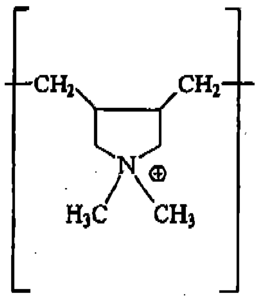

(III)

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9309759 A1
- WO 0193819 A1
- GB 1026978 A
- GB 1153196 A
- DE 2359399
- JP 02019576 A
- WO 9615765 A
- EP 998908 A2
- DE 3725030 A
- DE 3723354 A
- DE 3926344 A


### In der Beschreibung aufgeführte Nicht-Patentliteratur


- **CH. ZVIAK.** The Science of Hair Care. 248-250
- THE SCIENCE OF HAIR CARE. 264-267
- Dermatology. THE SCIENCE OF HAIR CARE. Verlag Marcel Dekker Inc, vol. 7
- Europäische Inventar der Kosmetik-Rohstoffe. THE SCIENCE OF HAIR CARE
- **KH. SCHRADER.** Grundlagen und Rezepturen der Kosmetika. Hüthig Buch Verlag, 1989